# FASCICULE DE BREVET EUROPEEN

(11) **EP 0 631 762 B1**
(45) Date de publication et mention de la délivrance du brevet: **31.07.2002**
(21) Numéro de dépôt: 94401147.7
(22) Date de dépôt: 25.05.1994
(51) Int. Cl.: A61F 2/06, A61F 2/04

(54) **Prothèse pour canal corporel**
Prothese für eine Körperröhre
Prosthesis for body duct

(30) Priorité: 24.06.1993 FR 9307693
(43) Date de publication de la demande: 04.01.1995
(73) Titulaire: PORGES, 92350 Le Plessis-Robinson (FR)
(72) Inventeur: Torchio, Gérard, F-91370 Verriere le Buisson (FR)
(74) Mandataire: Bonnetat, Christian

(56) Documents cités:
- EP-A- 0 481 365
- WO-A-89/01798
- WO-A-91/16005
- WO-A-92/19310
- WO-A-93/13824
- DE-A- 4 130 431
- DE-C- 321 666
- FR-A- 1 576 374
- FR-A- 2 661 603
- GB-A- 2 172 203
- US-A- 4 250 873
- US-A- 4 955 859
- US-A- 4 994 066

## Description

La présente invention concerne une prothèse destinée à être introduite dans un canal corporel comme définie dans le préambule de la revendication 1. Une telle prothèse est par exemple connue du document FR-A-2 661 603. On connaît ainsi une prothèse urétrale amovible pour le traitement des sténoses récidivantes dans l'urètre bulbo-membraneux. Cette prothèse a pour but de maintenir ouvert l'urètre pendant la cicatrisation après l'urétrotomie et sert ainsi de support à la cicatrisation de la paroi urétrale. Cette prothèse comporte deux parties, se présentant chacune sous forme d'un ressort hélicoïdal à spires jointives. Ces deux ressorts ont un même diamètre et des longueurs différentes et sont reliés l'un à l'autre par un fil métallique. Le ressort le plus long sert de prothèse proprement dite, maintenant l'urètre ouvert au diamètre du ressort, tandis que le ressort le plus court, relié à l'extrémité distale du ressort le plus long, permet d'immobiliser ce dernier en position par fixation dans la prostate. Toutefois, dans certains cas, cette prothèse a tendance à descendre dans l'urètre car la fixation dans la prostate par simple serrage n'est pas toujours suffisante, par exemple quand l'urètre prostatique est trop large. De plus, quand la prostate a été réséquée ou après une prostatectomie, une prothèse telle que définie ci-dessus ne peut pas être utilisée.

La présente invention a pour but d'éviter ces inconvénients, et concerne une prothèse adaptée pour pouvoir être immobilisée en position, de façon positive, dans un canal corporel.

A cet effet, présente invention propose une prothèse conformemmant à la revendication 1.

Ainsi, grâce à cette structure radialement déformable du dispositif de maintien, ce dernier peut être introduit, avec la prothèse proprement dite, sans problème dans le canal corporel et, lorsque la prothèse est mise en place, le dispositif de maintien immobilise en position, de façon positive, la prothèse (tube flexible), après être passé dans son second état, par appui et blocage sur le canal corporel au diamètre duquel il peut s'adapter.

Pour l' introduction dans le canal corporel, les spires oblongues du ressort peuvent être inclinées de sorte que leur grande dimension ne présente plus, dans le canal, qu'une "hauteur" correspondant au diamètre interne de ce dernier, y compris le tube d'introduction de la prothèse. Une fois la prothèse en place, les spires oblongues reviennent en position initiale, ce qui permet alors d'immobiliser positivement la prothèse dans le canal.

De préférence, les spires dudit ressort sont au moins sensiblement jointives.

Selon une première variante de cet exemple de réalisation, la section transversale oblongue dudit ressort est constante sur toute la longueur de ce dernier, tandis que, selon une seconde variante, la grande dimension de la section transversale oblongue dudit ressort croît progressivement de l'extrémité proximale à l'extrémité distale de ce dernier.

De plus, ledit ressort peut être réalisé en fil métallique de qualité médicale.

De plus, ledit tube flexible peut être un ressort hélicoïdal, à spires au moins sensiblement jointives, de section transversale circulaire, tandis que lesdits moyens de liaison peuvent être constitués par un fil métallique.

Les figures du dessin annexé feront bien comprendre comment l'invention peut être réalisée. Sur ces figures, des références identiques désignent des éléments semblables.

La figure 1 est une vue de côté d'un premier exemple de réalisation d'une prothèse urétrale selon l'invention.

La figure 2 est une vue semblable à la figure 1, mais tournée de 90° par rapport à cette dernière.

La figure 3 est une vue selon la flèche III de la figure 1.

La figure 4 montre la prothèse urétrale de la figure 1 insérée dans un tube de mise en place.

La figure 5 est semblable à la figure 4, mais avec le dispositif de maintien sorti du tube.

La figure 6 illustre la prothèse urétrale selon la figure 1, mise en place dans l'urètre d'un patient.

La figure 7 est une vue de côté d'une variante du premier exemple de réalisation d'une prothèse urétrale selon l'invention.

La figure 8 est une vue semblable à la figure 7, mais tournée de 90° par rapport à cette dernière.

La figure 9 est une vue selon la flèche IX de la figure 7.

La figure 10 montre la prothèse urétrale de la figure 7 insérée dans un tube de mise en place.

La figure 11 est semblable à la figure 10, mais avec le dispositif de maintien sorti du tube.

La figure 12 illustre la prothèse urétrale selon la figure 7, mise en place dans l'urètre d'un patient.

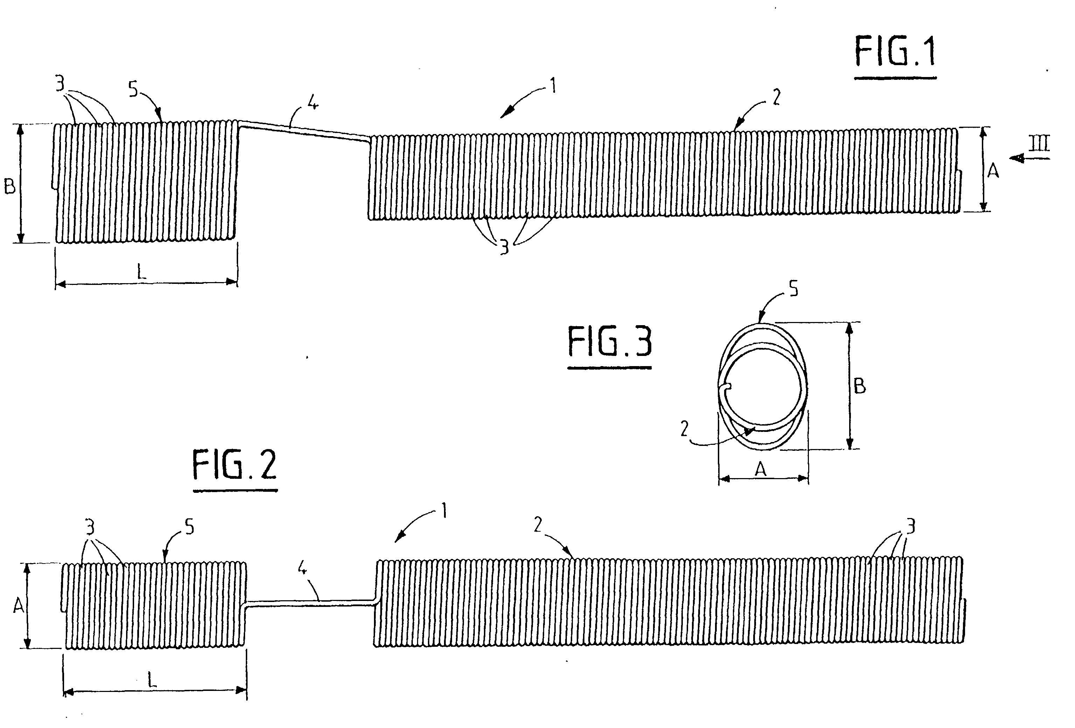

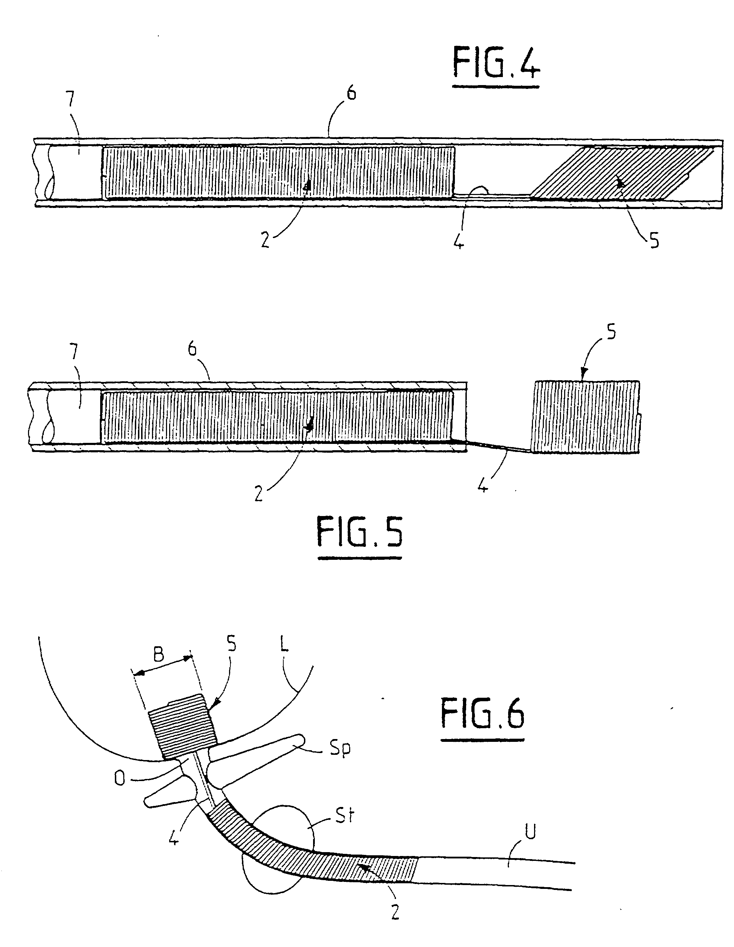

Sur la figure 1, on a représenté, en vue de côté, un premier exemple de réalisation d'une prothèse urétrale 1 conforme à l'invention. La prothèse 1 comporte une première partie 2 sous forme d'un premier ressort hélicoïdal à spires jointives 3, constituant la prothèse proprement dite, destinée à être logée dans l'urètre bulbo-membraneux U au niveau d'une sténose St (figure 6). A titre d'exemple non limitatif et pour donner un ordre de grandeur, le ressort 2 de section circulaire peut présenter un diamètre extérieur constant d'environ 6,6 mm et une longueur d'environ 60 mm. Le premier ressort 2 est relié, par l'intermédiaire d'un fil métallique 4, à un second ressort hélicoïdal 5 à spires jointives 3, destiné à former le dispositif de maintien du ressort 2 dans la prostate ou dans la loge prostatique L (figure 6). On notera que, comme représenté, le fil métallique 4, destiné à traverser le sphincter Sp (figure 6), et dont la longueur peut être de l'ordre de 10 mm, peut être formé en une seule pièce avec les premier et second ressorts et que, par ailleurs, le premier ressort 2, le fil métallique 4, et le second ressort 5 sont avantageusement réalisés en un métal inoxydable de qualité médicale.

Le second ressort 5 est plus court que le premier ressort 2 et peut présenter, par exemple, une longueur d'environ 15 mm. De plus, selon l'invention et contrairement au premier ressort 2 présentant une section transversale circulaire (figure 3), le second ressort 5 présente une forme oblongue en section transversale (figure 3), sensiblement elliptique ou en forme de "piste d'athlétisme". A titre d'exemple non limitatif, le grand "axe" A de cette "ellipse" peut présenter une longueur d'environ 10 mm, et son petit "axe" B une longueur d'environ 6,6 mm. On notera que, dans ce cas, cette section transversale oblongue est constante sur toute la longueur L du second ressort 5.

Comme le montre la figure 4, la prothèse 1 peut être introduite dans le tube de mise en place 6 (constituant un équipement standard d'un diamètre intérieur déterminé correspondant au diamètre extérieur du ressort 2) en inclinant les spires oblongues du ressort 5. Cela est possible du fait que la "largeur" de la section transversale oblongue du ressort 5 est égale au diamètre du ressort 2. Au moment de la mise en place de la prothèse dans l'urètre, à l'aide du poussoir 7, le ressort 5 "incliné" dans le tube reprend sa forme initiale en sortant de ce dernier (figure 5).

Comme on le voit sur la figure 6, une fois le tube de mise en place 6 retiré, la longueur B du grand "axe" de la section transversale du ressort 5 est suffisante pour assurer le maintien en position du ressort 2 dans l'urètre U au niveau de la sténose St, par appui sur le bord de l'ouverture 0 de l'urètre U débouchant dans la loge prostatique L.

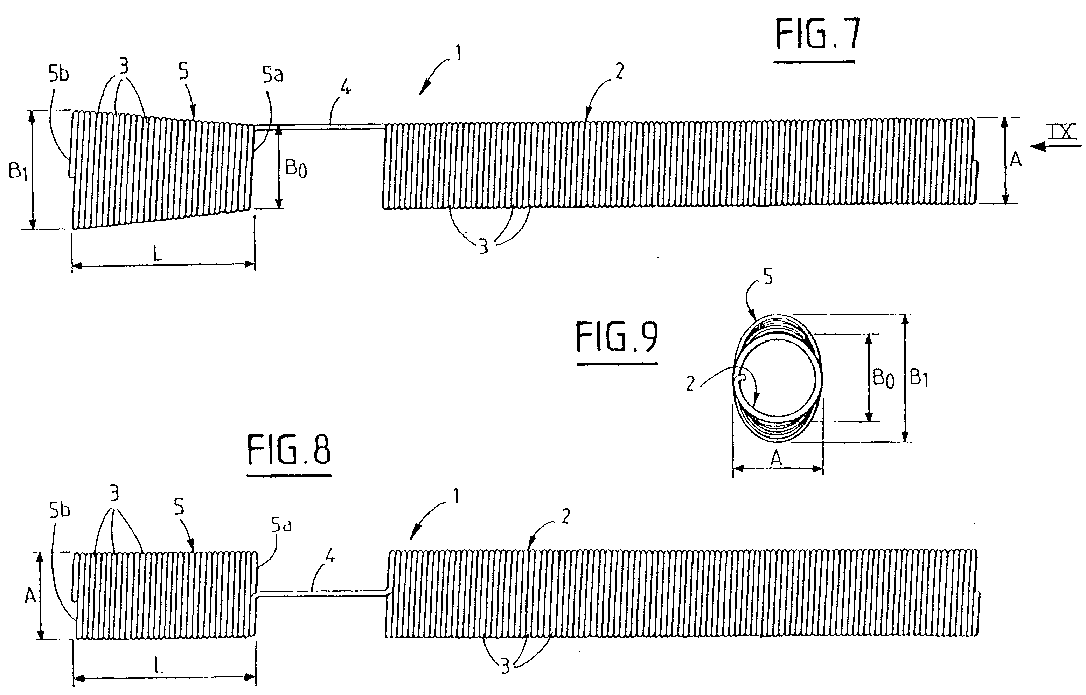

La variante du premier exemple de réalisation de la prothèse urétrale 1, montrée sur les figures 7 à 12, ne diffère de la prothèse des figures 1 à 6 que par le fait que, dans ce cas, le second ressort 5 présente une forme sensiblement tronconique de section transversale également sensiblement elliptique ou en forme de "piste d'athlétisme", mais dont le grand "axe" B croît progressivement (par exemple de 6,6 mm à 10 mm) de l'extrémité proximale 5a (Bo) du ressort 5 en regard du ressort 2 à son extrémité distale opposée 5b (B1), tandis que le petit "axe" A (égal au diamètre du ressort 2) reste constant sur toute la longueur L du ressort 5 (voir les figures 7 à 9). On notera qu'en fait, à l'extrémité 5a, Bo est égal à A.

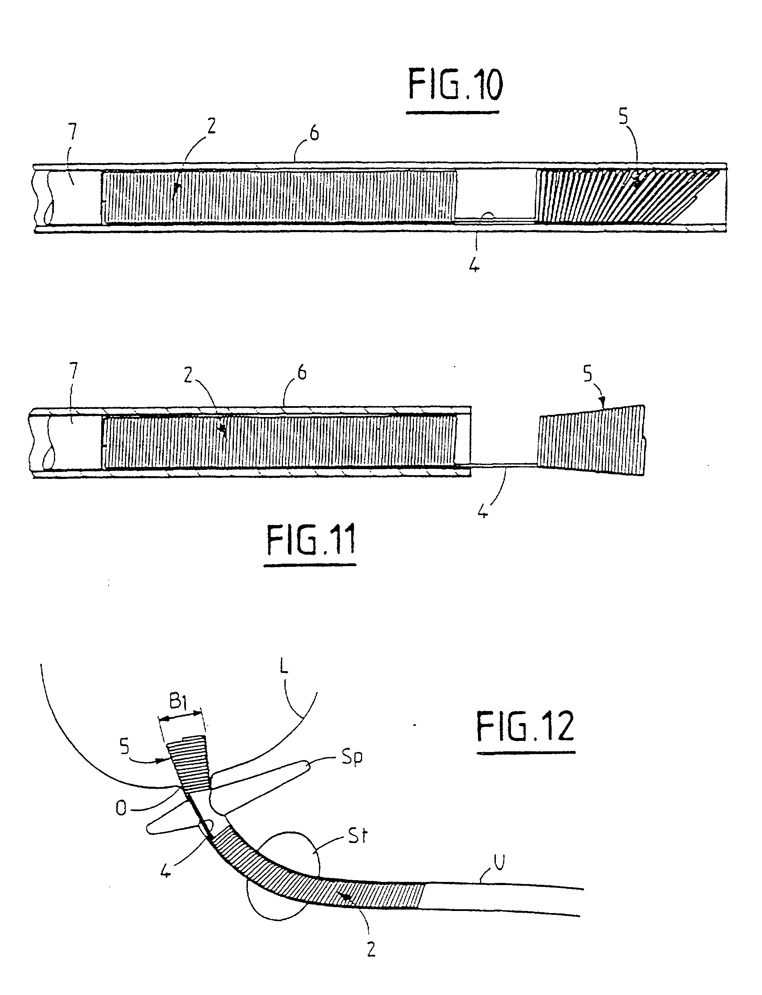

Cette configuration du second ressort 5 permet de même l'introduction de la prothèse 1 dans le tube de mise en place 6 (figure 10) en inclinant les spires oblongues du ressort 5, lequel reprend sa forme initiale en sortant du tube 6 sous l'action du poussoir 7 (figure 11).

Par ailleurs, comme on le voit sur la figure 12, le ressort 2 est alors maintenu en position dans l'urètre U au niveau de la sténose St, par appui du ressort 5 au voisinage de l'ouverture 0 de l'urètre U débouchant dans la loge prostatique L, la partie d'extrémité proximale du ressort 5 s'enfonçant légèrement dans l'urètre U jusqu'à être bloquée lorsque l'augmentation de la longueur B, vers l'extrémité distale du ressort 5, est suffisante.

## Revendications

1. Prothèse urétrale amovible pour le traitement d'une sténose de l'urètre bulbo-membraneux (U), comportant :
• un tube flexible (2) destiné à conserver la section normale de passage de l'urètre bulbo-membraneux (U) et constituant la prothèse proprement dite ;
• un dispositif de maintien (5) destiné à immobiliser en position ledit tube flexible (2) dans l'urètre bulbo-membraneux (U), ledit dispositif de maintien (5) étant radialement déformable et susceptible de passer d'un premier état dans lequel sa section transversale est au plus égale à celle de l'urètre (U), à un second état dans lequel sa section transversale est telle que ledit dispositif (5) peut prendre appui sur la paroi interne de l'urètre au voisinage du débouché de celui-ci dans une cavité (L) et, inversement, de passer dudit second état audit premier état ; et
• des moyens de liaison filaires (4) reliant ledit dispositif de maintien (5) à l'extrémité distale dudit tube flexible (2) en maintenant ledit dispositif de maintien (5) à distance de ladite extrémité, lesdits moyens de liaison filaires (4) étant destinés à traverser le sphincter,
**caractérisé en ce que** ledit dispositif de maintien est une structure tubulaire (5), constituée par un ressort hélicoïdal (5), présentant sur au moins la plupart de sa longueur une section transversale de forme oblongue, dont la petite dimension (A) est égale au diamètre du tube flexible (2) et au plus égale au diamètre intérieur de l'urètre bulbo-membraneux (U).

2. Prothèse selon la revendication 1,
**caractérisée en ce que** les spires (3) dudit ressort hélicoïdal (5) sont au moins sensiblement jointives.

3. Prothèse selon l'une des revendications 1 ou 2,
**caractérisée en ce que** la section transversale oblongue dudit ressort (5) est constante sur toute la longueur de ce dernier.

4. Prothèse selon l'une des revendications 1 ou 2,
**caractérisée en ce que** la grande dimension (B) de la section transversale oblongue dudit ressort hélicoïdal (5) croît progressivement de l'extrémité proximale (5a) à l'extrémité distale (5b) de ce dernier.

5. Prothèse selon l'une quelconque des revendications 1 à 4,
**caractérisée en ce que** ledit ressort (5) est réalisé en fil métallique de qualité médicale.

6. Prothèse selon l'une quelconque des revendications 1 à 5,
**caractérisée en ce que** ledit tube flexible est un ressort hélicoïdal (2), à spires (3) au moins sensiblement jointives, de section transversale circulaire.

7. Prothèse selon l'une quelconque des revendications 1 à 6,
**caractérisée en ce que** lesdits moyens de liaison filaires sont constitués par un fil métallique (4).

## Patentansprüche

1. Entfernbare Harnröhrenprothese zur Behandlung einer Stenose des bulbourethralen und membranösen Abschnitts der Harnröhre (U), die folgendes umfaßt:
- einen Schlauch (2), der zur Bewahrung des normalen Durchlaßquerschnitts des bulbourethralen und membranösen Abschnitts der Harnröhre (U) bestimmt ist und die eigentliche Prothese bildet;
- eine Haltevorrichtung (5) zur Festsetzung des Schlauchs (2) in Position in dem bulbourethralen und membranösen Abschnitt der Harnröhre (U), wobei die Haltevorrichtung (5) radial verformbar ist und aus einem ersten Zustand, in dem ihr Querschnitt höchstens gleich dem der Harnröhre (U) ist, in einen zweiten Zustand, in dem ihr Querschnitt derart ist, daß sich die Vorrichtung (5) an der Innenwand der Harnröhre in der Nähe von deren Mündung in eine Kavität (L) abstützen kann, und umgekehrt aus dem zweiten Zustand in den ersten Zustand übergehen kann; und
- Filamentverbindungsmittel (4), die die Haltevorrichtung (5) mit dem distalen Ende des Schlauchs (2) verbinden und dabei die Haltevorrichtung (5) in einem Abstand von dem Ende halten, wobei die Filamentverbindungsmittel (4) den Schließmuskel überqueren sollen,
**dadurch gekennzeichnet, daß** die Haltevorrichtung eine röhrenförmige Konstruktion (5) ist, die aus einer Schraubenfeder (5) besteht, weiche über zumindest den Großteil ihrer Länge einen länglichen Querschnitt aufweist, dessen kleine Abmessung (A) gleich dem Durchmesser des Schlauchs (2) und höchstens gleich dem Innendurchmesser des bulbourethralen und membranösen Abschnitts der Harnröhre (U) ist.

2. Prothese nach Anspruch 1,
**dadurch gekennzeichnet, daß** die Windungen (3) der Schraubenfeder (5) zumindest im wesentlichen aneinander angrenzen.

3. Prothese nach Anspruch 1 oder 2,
**dadurch gekennzeichnet, daß** der längliche Querschnitt der Feder (5) über deren gesamte Länge konstant ist.

4. Prothese nach Anspruch 1 oder 2,
**dadurch gekennzeichnet, daß** die große Abmessung (B) des länglichen Querschnitts der Schraubenfeder (5) vom proximalen Ende (5a) bis zum distalen Ende (5b) der letzteren allmählich zunimmt.

5. Prothese nach einem der Ansprüche 1 bis 4,
**dadurch gekennzeichnet, daß** die Feder (5) aus Metalldraht medizinischer Qualität hergestellt ist.

6. Prothese nach einem der Ansprüche 1 bis 5,
**dadurch gekennzeichnet, daß** der Schlauch eine Schraubenfeder (2) kreisrunden Querschnitts mit zumindest im wesentlichen aneinander angrenzenden Windungen (3) ist.

7. Prothese nach einem der Ansprüche 1 bis 6,
**dadurch gekennzeichnet, daß** die Filamentverbindungsmittel aus einem Metalldraht (4) bestehen.

## Claims

1. Removable urethral prosthesis for treating a stenosis of the bulbo-membranous urethra (U), comprising:
• a flexible tube (2) intended to preserve the normal cross-sectional passage of the bulbo-membranous urethra (U) and constituting the prosthesis proper;
• a holding device (5) intended to immobilize said flexible tube (2) in position in the bulbo-membranous urethra (U), said holding device (5) being radially deformable and capable of changing from a first state, in which its cross section is at most equal to that of the urethra (U), to a second state, in which its cross section is such that said device (5) can bear on the inner wall of the urethra near to where the latter opens into a cavity (L), and, conversely, of changing from said second state to said first state; and
• filamentary connection means (4) connecting said holding device (5) to the distal end of said flexible tube (2), maintaining said holding device (5) at a distance from said end, said filamentary connection means (4) being intended to pass through the sphincter,
**characterized in that** said holding device is a tubular structure (5) formed by a helical spring (5) having along at least most of its length a cross section of oblong shape, of which the small dimension (A) is equal to the diameter of the flexible tube (2) and at most equal to the internal diameter of the bulbo-membranous urethra (U).

2. Prosthesis according to Claim 1, **characterized in that** the windings (3) of said helical spring (5) are at least substantially contiguous.

3. Prosthesis according to one of Claims 1 and 2,
**characterized in that** the oblong cross section of said spring (5) is constant along the entire length of the latter.

4. Prosthesis according to one of Claims 1 and 2,
**characterized in that** the large dimension (B) of the oblong cross section of said helical spring (5) increases progressively from the proximal end (5a) to the distal end (5b) of the latter.

5. Prosthesis according to any one of Claims 1 to 4, **characterized in that** said spring (5) is made of metal wire of medical quality.

6. Prosthesis according to any one of Claims 1 to 5, **characterized in that** said flexible tube is a helical spring (2), with at least substantially contiguous windings (3), and with a circular cross section.

7. Prosthesis according to any one of Claims 1 to 6, **characterized in that** said filamentary connection means are formed by a metal wire (4).
